# EUROPEAN PATENT SPECIFICATION

(11) **EP 2 560 644 B1**
(45) Date of publication and mention of the grant of the patent: **29.06.2016**
(21) Application number: 11736457.0
(22) Date of filing: 18.04.2011
(51) Int. Cl.: A61K 31/4365, A61K 31/60, A61K 38/18, A61P 13/12, A61K 45/06, A61K 31/381, A61K 31/616

(54) **A PHARMACEUTICAL COMPOSITION COMPRISING ANTIPLATELET AGENTS AND AN ERYTHROPOIESIS STIMULATING AGENT**
PHARMAZEUTISCHE ZUBEREITUNG ENTHALTEND THROMBOZYTENHEMMER UND EINE ERYTHROPOIESE STIMULIERENDE VERBINDUNG
COMPOSITION PHARMACEUTIQUE COMPRENANT DES AGENTS ANTIPLAQUETTAIRES ET UN AGENT STIMULANT L'ÉRYTHROPOÏÈSE

(30) Priority: 19.04.2010 IN MU12812010
(43) Date of publication of application: 27.02.2013
(73) Proprietor: Cadila Healthcare Limited, Ahmedabad 380 015 Gujarat (IN)
(72) Inventor: SONI, Hitesh, Madanlal, Ahmedabad 380 015 Gujarat (IN); JAIN, Mukul, R., Ahmedabad 380 015 Gujarat (IN)
(74) Representative: Zacco Denmark A/S
(86) International application number: PCT/IN2011/000259
(87) International publication number: WO 2011/132201

(56) References cited:
- US-A1- 2006 094 648
- US-B1- 6 521 245
- TAYLOR J E ET AL: "ERYTHROPOIETIN AND SPONTANEOUS PLATELET AGGREGATION IN HAEMODIALYSIS PATIENTS", LANCET, vol. 338, no. 8779, 1991, pages 1361-1362, XP002659824, ISSN: 0099-5355
- KOOISTRA M P ET AL: "Low-dose aspirin does not prevent thrombovascular accidents in low-risk haemodialysis patients during treatment with recombinant human erythropoietin", NEPHROLOGY DIALYSIS TRANSPLANTATION, OXFORD UNIVERSITY PRESS, GB, vol. 9, no. 8, 1 January 1994 (1994-01-01) , pages 1115-1120, XP009090353, ISSN: 0931-0509
- MESQUITA MARIA DO CARMO FILOMENA ET AL: "Management of Refractory Essential Thrombocythemia With Anagrelide in a Patient Undergoing Hemodialysis", CLINICAL THERAPEUTICS, vol. 31, no. 11, November 2009 (2009-11), pages 2559-2564, XP026872257, ISSN: 0149-2918
- TANG Y D ET AL: "Effects of recombinant human erythropoietin on platelet activation in acute myocardial infarction: Results of a double-blind, placebo-controlled, randomized trial", AMERICAN HEART JOURNAL, MOSBY- YEAR BOOK INC, US, vol. 158, no. 6, 1 December 2009 (2009-12-01), pages 941-947, XP026787882, ISSN: 0002-8703, DOI: 10.1016/J.AHJ.2009.06.032 [retrieved on 2009-12-01]

## Description

### FIELD OF THE INVENTION

The present invention is related to a composition of antiplatelet agents and erythropoiesis stimulating agents suitable for chronic kidney disorders.

### BACKGROUND OF THE INVENTION

The present invention is related to a composition of suitable erythropoiesis stimulating agents (ESAs) and antiplatelet agents for the reduction of progression of kidney damage due to the use of erythropoiesis stimulating agent in Chronic Kidney Disease (CKD). Further, the present invention is related to a combination of ESAs and antiplatelet agents to reduce the platelet reactivity induced by erythropoiesis stimulating agent in CKD and to reduce the bleeding problem associated with antiplatelet agent, which eventually reduces the cardiovascular risk associated with ESAs in CKD.

Chronic kidney disorder is related to progressive and irreversible decline in the kidney function and considered to be a risk factor for cardiovascular disease. Reduction in kidney function leads to maladaptive changes in fluid retention (extracellular fluid overload), anemia, disturbances of bone and mineral metabolism and dyslipidemia. Indeed, patients with reduced kidney function represent a population not only at risk for progression of kidney disease and for end-stage renal disease (ESRD), but also at even greater risk for cardiovascular diseases. At present, there are no specific cures for reduction of the progression of chronic kidney diseases, and renal transplantation is limited by organ shortage, therefore present composition is useful for the prevention of progression of renal diseases. t is found that treatment with recombinant human erythropoietin (rHuEPO) accelerates the progression of renal diseases (Garcia DL et al, PNAS, 6142-6146, 1988). Moreover rHuEPO also augments the platelet reactivity that makes the patient susceptible to cardiovascular disease and thrombotic events. Therefore it appears that when the chronic kidney patient is treated with rHuEPO it increases both the impairment of kidney functions and the risk of cardiovascular coronary thrombosis.

Cardiovascular disease is very common health problem, which is the major cause of death. Antiplatelet agents are one among the known treatment agents for cardiovascular disorders. There are several antiplatelet agents known like clopidogrel, prasugrel etc. The structure and their functions are known from US4847265, US6429210 and US6693115 respectively.

Clopidogrel is marketed as its bisulfate salt under the brand name Plavix, which is in solid form. Several solid formulations of clopidogrel are known but because of low solubility of clopidogrel bisulfate, it is difficult to prepare its injectable formulation. In fact, there is no injectable formulation available in the market for clopidogrel bisulfate.

These antiplatelet agents are associated with some side effects like it may take longer for bleeding to stop if one experiences a cut or injury, while taking these agents. Clopidogrel (Plavix) and prasugrel both are associated with this problem *(Sanofi-Aventis, BMS, www.plavix.com and* NEJM Volume 361:940-942*)*

Combination of clopidogrel and aspirin has better effect in treatment of acute coronary syndrome, but it has been found that there is high risk of increase in bleeding time on administration of this combination. This problem has been observed in hemodialysis population also, when the combination of clopidogrel and aspirin is administered. *(*NEJM vol345 No7, 2001. 494-502 *and* J Am Soc Nephrol 14: 2313-2321, 2003*)*

Patients on hemodialysis or chronic kidney disorders face other complications, which mainly include anemia and cardiovascular disorders. For the treatment of anemia, it is necessary to administer erythropoietin to patients of chronic kidney disorders.

Erythropoietin (EPO), mainly produced by the peritubular cells of the kidney is the primary regulator of red blood cell production and is responsible for the terminal differentiation of erythroid progenitor cells.

In bone marrow, EPO acts on a specific receptor (EPO-R), with subsequent activation of various signaling pathways (STAT5, MAPK, PI3/Akt). EPO acts primarily as a survival factors for erythroid progenitor cells, and in this manner increases the number of mature red blood cells in the circulation.

At present, Recombinant Human Erythropoietin (rHuEPO) is approved for treatment of anemia caused by conditions, including anemia associated with renal failure, chemotherapy, and HIV antiviral treatment or to reduce the need for transfusion in preoperative surgical patients. It is also one of the products used for the treatment of aplastic anemia. Recombinant Human Erythropoietin (rHuEPO) should be started if patients Hb remains below 11gm% despite correcting the nutritional and iron deficiencies. The intravenous route is recommended in patients on hemodialysis while in others it can be used subcutaneously two to three times a week.

A study in nephrectomized rat showed that correction of anemia with rHuEPO may have adverse renal hemodynamic and structural consequences *(*Garcia DL et al, PNAS, 6142-6146, 1988*).*

A study in dogs suggested that rHuEPO not only promotes the synthesis of increased numbers of reticulated platelets but these newly produced platelets are hyper reactive compared with controls. *(*Thromb haemost 1997; 78:1505-09, Blood 2000; 95:2983-89). These newly produced platelets increase platelet reactivity and are linked to an increased risk of thrombotic events. EPO seems to affect various cardiovascular and haemostatic parameters unrelated to increase in haematocrit *(*Blood 2000; 95:2983-89). EPO may also be prothrombotic in humans both by increasing platelet reactivity and by increasing systemic BP through vasoconstriction. It also increased calcium uptake and store in platelets. *(*Curr Opin Nephrol Hypertens 2001; 10:633-77).

EPO increases the red blood cells significantly in patients which may cause serious risk of venous thrombosis (Red clot) and therefore heparin and low molecular weight heparin (LMWH) are preferred medicament for the treatment of red clot. In addition, another drawback of rHuEPO is that it increases the platelet count and make them hyper-reactive which may lead to formation of platelet rich clot (white clot) in coronary artery that causes myocardial infarction in patient. However, the rHuEPO does not show any suitable therapeutic and preventive effect over the white clot. (Curr Opin Nephrol Hypertens 2001; 10:633-77, Blood 2000; 95:2983-89, BMJ 1990; 300:573-8, N Engl J Med 1998; 339:584-90, Cancer 2003; 9:1514-20, Clin Invest 1994; 72:S36-S43, Lancet 2007; 369:381-8, Clin J Am Soc Nephrol 2007; 2:1274-82, Am J Hypertension 2006; 19:1286-1292, PNAS USA 1988; 85, 6142-6146).

Thus, in a nutshell the problem associated with the administration of rHuEPO is that it not only increases the impairment of kidney function but also make the patient vulnerable to cardiovascular disease. These drawbacks are very severe and complicated which lead to high mortality rate in patient suffering from kidney diseases. Unfortunately, no therapy is available yet to prevent the rHuEPO-induced kidney damages and/or to improve in kidney function.

Hence, it is desirable to develop a composition which significantly improve the kidney function and also prevent further damage caused by rHuEPO and /or lower down the thrombotic events and bleeding problems in patients and also improve the renal function in patient.

### SUMMARY OF INVENTION

The invention is defined in the claims. The present invention provides a composition comprising of a suitable antiplatelet agent/agents or its pharmaceutical acceptable salts and a suitable erythropoiesis stimulating agent for the improvement in renal function. Specifically, such a composition provides therapeutic benefit by prevention and treatment of kidney damage and cardiovascular diseases in chronic kidney diseases patient. Furthermore, when administrated simultaneously, sequentially or separately, the suitable antiplatelet agent/agents and an erythropoiesis stimulating agent interact in a synergistic manner to control the problems associated with rHuEPO and improves the renal function.

Also described is a kit for the treatment of cardiovascular disorders and chronic kidney disorders, which comprises suitable antiplatelet agent/agents and an erythropoietin stimulating agent.

In a preferred feature, the present invention provides a liquid formulation of clopidogrel for intravenous administration so that it can be administered simultaneously with erythropoietin-stimulating agent.

### EMBODIMENTS OF THE INVENTION

In an embodiment, the present invention provides a composition comprising suitable anti-platelet agent or its pharmaceutical acceptable salts combined with suitable erythropoiesis stimulating agent for the treatment and improvement of impaired kidney function. Preferably the compounds are provided in a suitable ratio depending on the need of the patient.

In another embodiment, the invention provides composition of suitable antiplatelet agent or its pharmaceutical acceptable salts combined with suitable erythropoiesis stimulating agent for the improvement in kidney damage caused by administration of rHuEPO.

In a preferred embodiment, the present invention provides a composition comprising clopidogrel or its pharmaceutical acceptable salts in combination with recombinant human erythropoietin for the improvement in kidney damage caused by administration of rHuEPO and.

Disclosed herein is a composition of clopidogrel or its pharmaceutical acceptable salts in combination with recombinant human erythropoietin for the treatment of cardiovascular disorders wherein the bleeding problem associated with clopidogrel or its pharmaceutical acceptable salt and the risk of thrombotic events associated with recombinant human erythropoietin are reduced.

Disclosed herein is a combination of more than one antiplatelet agent and a suitable erythropoiesis stimulating agent for the treatment of one or more conditions described above.

Disclosed herein is further used for the curative, prophylactic treatment of chronic kidney disorders as well as conditions associated with chronic kidney disorders where the risk of thrombotic events associated with recombinant human erythropoietin is reduced.

In a further embodiment, the combination of the present invention comprising of a suitable antiplatelet agent or its pharmaceutical acceptable salts and erythropoiesis stimulating agents may be administrated by oral, intravenous or subcutaneous route of administration.

Disclosed herein is a kit comprising antiplatelet agent/agents with erythropoiesis stimulating agent.

### Brief description of drawings:

Fig 1a- Effect of rHuEPO on platelet reactivity in ADP-induced ex vivo platelet aggregation method using male Wistar rats.
Fig 1b- Effect of rHuEPO on platelet reactivity in arachidonic acid-induced ex vivo platelet aggregation method using male Wistar rats.
Fig 1c- Effect of rHuEPO on platelet reactivity in thrombin-induced ex vivo platelet aggregation method using male Wistar rats.
Fig 1d- Effect of rHuEPO on platelet reactivity in collagen-induced ex vivo platelet aggregation method using male Wistar rats.
Fig 2a- Effect of clopidogrel or aspirin or their combination on rHuEPO-induced platelet reactivity in ADP-induced ex vivo platelet aggregation method using male Wistar rats.
Fig 2b- Effect of clopidogrel or aspirin or their combination on rHuEPO-induced platelet reactivity in arachidonic acid-induced ex vivo platelet aggregation method using male Wistar rats.
Fig 2c- Effect of clopidogrel or aspirin or their combination on rHuEPO-induced platelet reactivity in thrombin-induced ex vivo platelet aggregation method using male Wistar rats.
Fig 2d- Effect of clopidogrel or aspirin or their combination on rHuEPO-induced platelet reactivity in collagen-induced ex vivo platelet aggregation method using male Wistar rats.
Fig 3 Alteration of bleeding time in Wistar rats when clopidogrel, aspirin and rHuEPO are used alone or in combination with each other.
Fig 4- Effects of various treatment on ex vivo % platelet aggregation using 2.5µM ADP as aggregating agent in chronic kidney disease model in rat.
Fig 5a- Effects of various treatment on RBC count (10⁶/µL) in chronic kidney disease model in rat.
Fig 5b- Effects of various treatment on Hemoglobin (Hb) (g/dL) in chronic kidney disease model in rat.
Fig 5c- Effects of various treatment on % Hematocrit (HCT) in chronic kidney disease model in rat.
Fig 6a- Effects of various treatment on Serum Creatinine (mg/dL) in chronic kidney disease model in rat.
Fig 6b- Effects of various treatment on Serum Urea (mg/dL) in chronic kidney disease model in rat.
Fig 6c- Effects of various treatment on Serum Uric acid (mg/dL) in chronic kidney disease model in rat.
Fig 6d- Effects of various treatment on Blood Urea Nitrogen (BUN) (mg/dL) in chronic kidney disease model in rat.
Fig 6e- Effects of various treatment on Total Protein (g/dL) in chronic kidney disease model in rat.

### DETAILED DESCRIPTION OF THE INVENTION

According to the present invention, a composition is provided comprising suitable antiplatelet agent/agents or their suitable pharmaceutically acceptable salts and an erythropoietin-stimulating agent for the curative, prophylactic treatment of chronic kidney diseases. Disclosed herein is a composition comprising suitable anti-platelet agent/agents and an erythropoietin-stimulating agent for the curative, prophylactic treatment of cardiovascular diseases.

Thus the present invention provides a composition comprising suitable anti-platelet agent or its pharmaceutical acceptable salts combined with suitable erythropoiesis stimulating agent for the treatment and improvement of impaired kidney function. Preferably the compounds are provided in a suitable ratio depending on the need of the patient.

In a preferred embodiment the invention provides composition comprising a suitable antiplatelet agent or its pharmaceutical acceptable salts combined with suitable erythropoiesis stimulating agent for the improvement in kidney damage caused by administration of rHuEPO.

The "antiplatelet agent" according to present invention is an agent which decreases the platelet aggregation. It may be selected from, but not limited to, clopidogrel and its pharmaceutically acceptable salt, prasugrel and its pharmaceutically acceptable salt and aspirin.

"Erythropoiesis stimulating agent" according to present invention is an agent, which stimulate the erythropoiesis. Erythropoiesis stimulating agent according to present invention may include recombinant human erythropoietin, darbepoetin, PEG-recombinant human erythropoietin, PEG- darbepoetin and other short peptides as well as small molecules etc. which stimulates erythropoiesis.

"Cardiovascular disorders" is well known in the art and can be described as the disorders associated with arteries and veins, which may include angina, atherosclerosis, cerebrovascular accidents, cerebrovascular diseases, congestive heart failure, coronary artery diseases, myocardial infarction, peripheral vascular diseases and cardiovascular diseases associated with chronic kidney diseases.

"Chronic kidney diseases" includes the diseases related to renal function and disorders associated with that and the diseases associated with chronic kidney disorders as given in for e.g. NEJM Volume 351:1296-1305. Chronic kidney disease in the general population is generally characterized by the existence of increased serum creatinine, serum urea, uric acid, blood urea nitrogen and decreased serum total protein and these can be used as kidney biomarkers. Worsening of renal function is defined as increase in about 25% of serum creatinine which roughly corresponds to a decrease in glomerular filtration rate (GFR) ≥ 9.0 ml/min/1.73 m². (Goldstein SL. Kidney International, 2010: 78, 433-435; A Leelahavanichkul et al. Kidney International, 2010: doi:10.1038/ki.2010.287*;* Damman K et al., Eur J Heart Fail, 2007: 6,17-18*;* Khan NA et al., J Am Soc Nephrol, 2006:17, 244-253*;* Krumholz HM et al., Am J Cardiol, 2000:85,1110-1113*).*

In an embodiment, the present invention provides improved therapeutic benefits and safety when a combination comprising anti-platelet agents and rHuEPO is used. In addition, the present combination of antiplatelet agents or their pharmaceutically acceptable salts with rHuEPO reduces the occurrence of problems associated with each other and acts complimentary to each other with better safety benefits. For example, rHuEPO has been linked with increased risk of thrombotic events because of hyper-reactivity of the platelets. On the other hand antiplatelet agent, for example, clopidogrel or its pharmaceutically acceptable salt, specifically those which are ADP-receptor antagonist, has been associated with increased risk of bleeding and causing aplastic anemia as well as bone marrow depression.

Pharmaceutically acceptable salts of clopidogrel which can be used are selected from bisulfate, besylate, tosylate, hydrochloride, hydrobromide and mesylate. In a preferred embodiment the pharmaceutically acceptable salts used may be clopidogrel besylate, clopidogrel bisulfate, clopidogrel hydrochloride and clopidogrel hydrobromide. Surprisingly, when used in combination, rHuEPO takes care of bleeding complications of anti-platelet agents such as clopidogrel; while anti-platelet agent/agents improves kidney functions which are impaired by the administration of rHuEPO as well as rHuEPO-induced hyper-reactivity of platelet therefore reducing the risk of thrombotic events.

Unless otherwise mentioned, in the specification whenever the term antiplatelet agent/agents or clopidogrel, prasugrel etc as well as Erythropoiesis stimulating agents selected from recombinant human erythropoietin, darbepoetin, PEG-recombinant human erythropoietin, PEG- darbepoetin are used, the term where applicable, also includes their suitable pharmaceutical composition.

In another embodiment a mechanistically different anti-platelet agent such as aspirin is used in combination with rHuEPO which also shows synergistic interaction and improves the kidney function caused by administration of rHuEPO in varying degrees. In another embodiment aspirin is used in combination with rHuEPO which shows synergistic interaction and normalizes the platelet reactivity-induced by rHuEPO with better safety in terms of bleeding. In another embodiment is provided a combination of rHuEPO with one or two mechanistically different anti-platelet agents for the curative and prophylactic treatment of chronic kidney disease patients. Disclosed herein is a combination of one or more suitable antiplatelet agents with suitable erythropoiesis stimulating agent, for the treatment of cardiovascular disorders and chronic kidney disorders.

In an embodiment, the combination of one or more antiplatelet agent/agents and erythropoietin stimulating agent used in said combination may be formulated for oral, parenteral or injectable administration. Injectable administration includes intravenous, intramuscular or subcutaneous injection.

This combination of antiplatelet agent/agents and recombinant human erythropoietin may be given either simultaneously or sequentially or at different time intervals as per requirement of dosing. The combination of the invention can be administered alone but one or both elements will generally be administrated with suitable pharmaceutical excipients, diluents or carrier selected with regard to intended route of administration as are known in the art.

In a preferred embodiment, the anti-platelet agents such as clopidogrel or its pharmaceutically acceptable salt such as clopidogrel bisulfate, clopidogrel besylate, clopidogrel hydrochloride and clopidogrel hydrobromide is administrated either orally or through parenteral route.

In an embodiment, the Clopidogrel salts can be given twice a day or once a day preferably, once a day. The dosage amount of clopidogrel or its pharmaceutically acceptable salt such as clopidogrel besylate is in the range from 5 mg to 600 mg, more preferably less than or equal to 75 mg in patients either given in a single dose or divided in multiple doses depending on the need of the patient. , it will be appreciated that the amount of dose will also depend on the severity of disease condition in patient.

In an embodiment the antiplatelet agent aspirin administrated orally or through parenteral route. Aspirin can be given twice a day or once a day, preferably once a day. The dosage amount of aspirin is in the range from 25 to 100 mg in the patient. However, the amount of dose depends over the severity of disease condition in patient.

In another embodiment the erythropoiesis stimulating agent such as rHuEPO is administrated through injection. The rHuEPO can be given oncea month or thrice a week or once a week, preferably thrice a week. However, the dosage administration frequency depends over the pharmacokinetic profile of erythropoiesis stimulating agent, the erythropoiesis stimulating agent used, severity of disease, patient profile etc. Preferably, the dosage amount of rHuEPO which may be used is in the range from 0.5µg/kg to 50µg/kg more preferably 0.5µg/kg to 2µg/kg. The dosage may be varied depending upon the requirements of the patient, the severity of the condition being treated.

In another embodiment, the suitable therapeutic amount of antiplatelet agent used in the combination is at least sufficient to reduce blood clotting. The suitable therapeutic amount of erythropoiesis stimulating agent used in the combination is at least sufficient to start erythropoiesis.

Disclosed herein is a kit for the treatment of one or more of disease conditions referred elsewhere in the specification. The Kit comprises as active ingredients a pharmaceutically effective amount of suitable antiplatelet agent/agents and an erythropoiesis stimulating agent. The active ingredients are formulated in same or different unit dosage form for different or same route of administration. The Kit comprises suitable anti-platelet agent/agents, which are formulated as oral, parenteral or injectable form and an erythropoiesis stimulating agent, which is formulated in an injectable form. Preferably, the antiplatelet agent/agents used in the kit are formulated to be administered by injection.

The present invention can be further illustrated with the help of following examples which disclose some of the embodiments of carrying out the invention and should not be considered as restriction on the scope of the invention.

### Example 1: Platelet reactivity enhanced by erythropoietin treatment

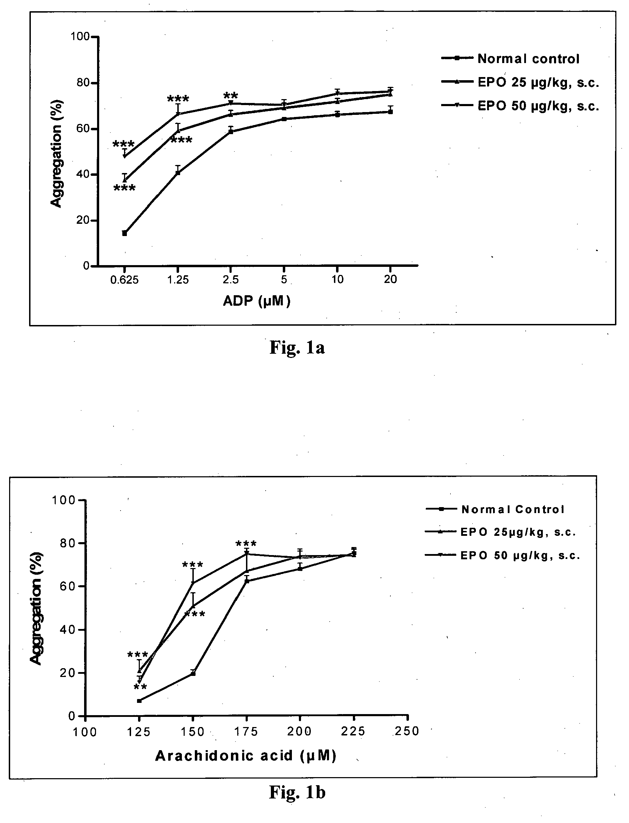

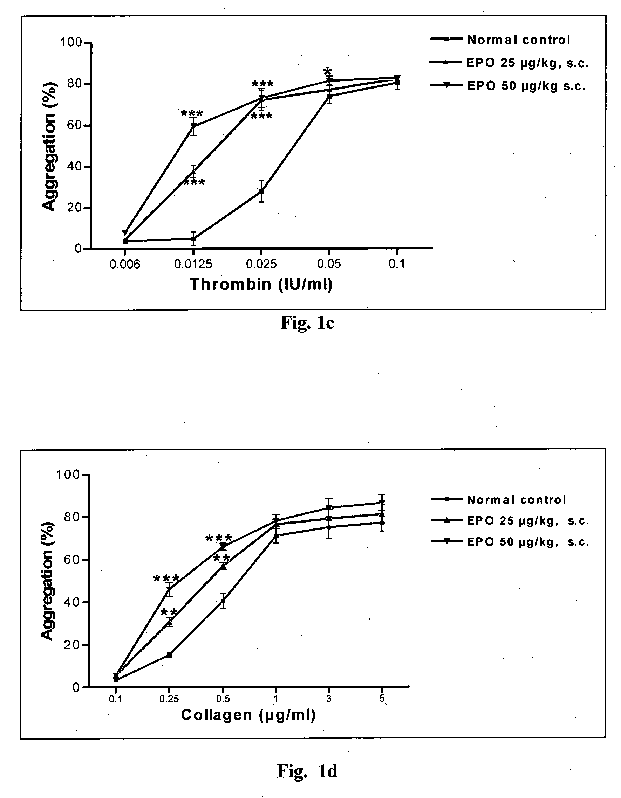

Six to eight week old male Wistar rats used and the methods and procedures described here have been reviewed and approved by the Institutional Animal Ethics Committee (IAEC). Wistar male rats were divided in groups [each group containing 6 animals (n=6)]. Control animal received vehicle only (0.9 % w/v NaCl normal saline), treatment groups received three consecutive doses of Recombinant Human Erythropoietin (25 and 50 µg/kg, s.c.) at an interval of 24 hour. Blood collection was performed at 48 hour after the last injection of Erythropoietin in citrated tubes. Platelet rich plasma (PRP) was separated. Determination of ex-vivo platelet aggregation was performed by using 1) ADP as aggregation inducing agent 2) Arachidonic acid as aggregation inducing agent 3) Thrombin as aggregation inducing agent and 4) Collagen as aggregation inducing agent. Platelet aggregation was performed by turbidometric method using high through put 96-well plate. Optical density was measured at 405 nM by Spectra Max plate reader. We found that rHuEPO causes increase in platelet aggregation. Increase in platelet aggregatory response meaning increase in platelet reactivity when same concentration of aggregating agent is used. When constant concentration of aggregating agents like ADP (1.25 µM), arachidonic acid (150 µM), thrombin (0.0125 IU/ml) and collagen (0.25 and 0.5 µg/ml) are used, platelet aggregation increases on increasing concentration of rHuEPO (25µg/kg, s.c and 50µg/kg, s.c (Fig 1a, 1b, 1c and 1d). Results are summarized in Table 1.

**Table 1: Effect of rHuEPO on platelet reactivity**

| Aggregating agents used | % platelet aggregation | | |
|---|---|---|---|
| | In Normal Rats | In rHuEPO (25µg/kg, s.c.) treated rats | In rHuEPO (50µg/kg, s.c.) treated rats |
| ADP (1.25 µM) | 40.6 ± 3.2 | 59.1 ± 3.2 | 66.4 ± 4.5 |
| Arachidonic acid (150 µM) | 19.4 ± 1.0 | 50.6 ± 3.1 | 61.3 ± 3.4 |
| Thrombin (0.0125 IU/ml) | 14.8 ± 0.6 | 37.7 ± 3.0 | 59.5 ± 4.4 |
| Collagen (0.25 µg/ml) | 15.0 ± 1.1 | 30.5 ± 2.0 | 45.9 ± 3.2 |
| Collagen (0.5 µg/ml) | 40.4 ± 3.6 | 56.9 ± 1.7 | 65.9 ± 1.7 |

### Example 2: Reduction of rHuEPO -induced hyper reactivity of platelets in presence of antiplatelet agents

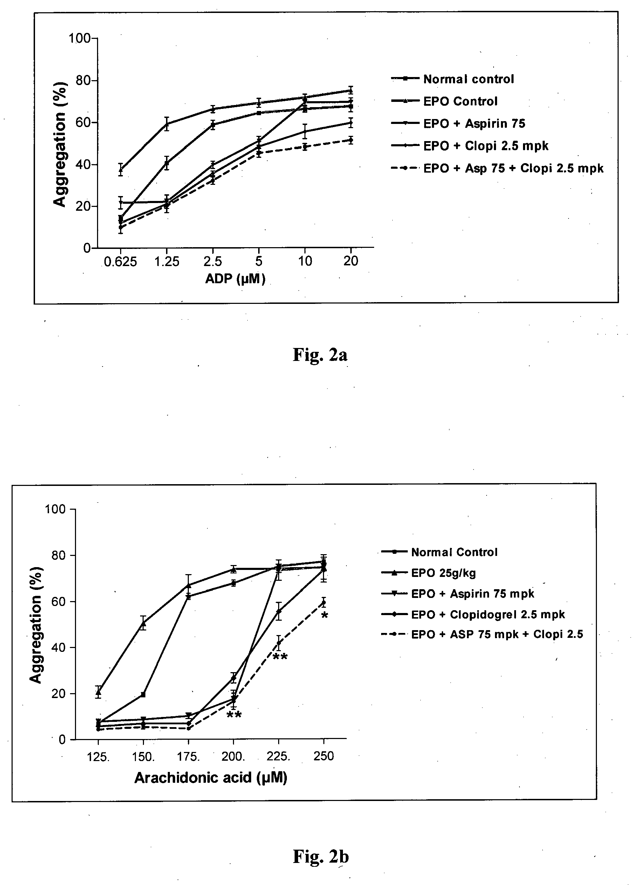

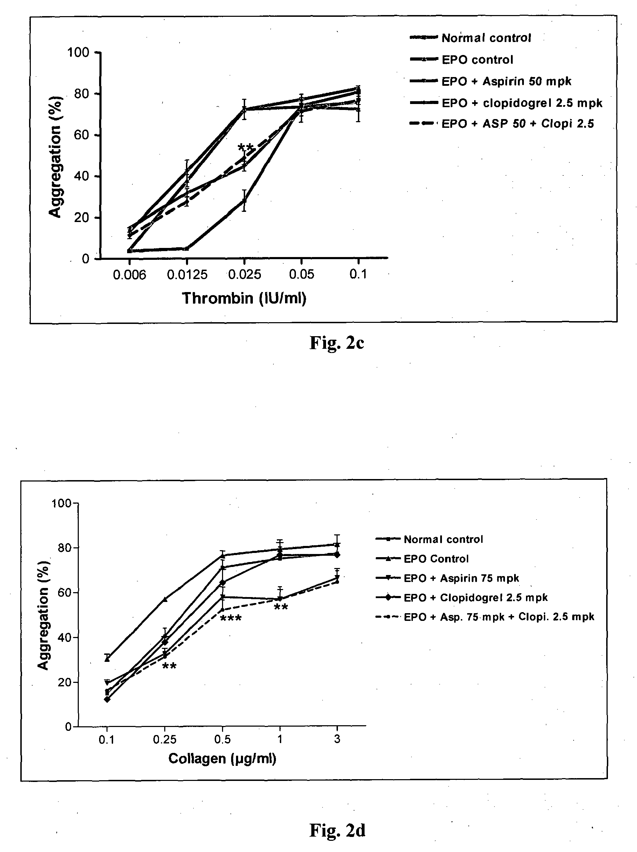

Six to eight week old male Wistar rats were divided in groups [each group containing 6 animals (n=6)]. Control animal received vehicle only (0.9 % w/v NaCl normal saline), treatment groups received three consecutive doses of Recombinant Human Erythropoietin (25 µg/kg, s.c.) at an interval of 24 hour. Aspirin (75mg/kg p.o.), and Clopidogrel (2.5mg/kg, p.o.) were dosed individually and in combination with each other to respective groups at 48 hour after the last injection of erythropoietin. Blood collection was performed after 2 hours of antiplatelet treatment. Platelet rich plasma (PRP) was separated. Determination of ex-vivo platelet aggregation was performed by using 1) ADP as aggregation inducing agent 2) Arachidonic acid as aggregation inducing agent 3) Thrombin as aggregation inducing agent and 4) Collagen as aggregation inducing agent. When constant concentration of aggregating agents like ADP (1.25 µM), arachidonic acid (150 µM), thrombin (0.0125 IU/ml) and collagen (0.25 and 0.5 µg/ml) are used, on administration of clopidogrel (2.5 mg/kg p.o.) to rHuEPO (25µg/kg, S.C.) treated rats, platelet aggregatory response is normalized (Fig 2a, 2b, 2c and 2d). Administration of aspirin (75 mg/kg/p.o.) to these animals further normalizes the hyper reactive platelets. Results are summarized in table 2.

**Table 2: Effect of Clopidogrel and Aspirin on rHuEPO induced platelet reactivity**

| Aggregating agents used | % platelet aggregation | | | |
|---|---|---|---|---|
| | In Normal Rats | In rHuEPO (25µg/kg, s.c.) treated rats | rHuEPO (25µg/kg, s.c.) and Clopidogrel (2.5 mg/kg p.o.) | rHuEPO (25µg/kg, s.c.) and Clopidogrel (2.5 mg/kg p.o.) and Aspirin (75 mg/kg/p.o.) |
| ADP (1.25 µM) | 39.3 ± 2.5 | 59.5 ± 1.4 | 24.1 ± 1.3 | 22.6 ± 1.2 |
| Arachidonic acid (150 µM) | 18.25 ± 1.7 | 44.4 ± 2.5 | 8.5 ± 0.5 | 6.5 ± 0.2 |
| Thrombin (0.025 IU/ml) | 27.7 ± 2.3 | 70.8 ± 1.0 | 49.52 ± 2.51 | 36.69 ± 1.48 |
| Collagen (0.25µg/ml) | 40.6 ± 1.7 | 60.03 ± 1.9 | 36.57 ± 0.9 | 24.66 ± 1.0 |

### Example 3: Reduction in bleeding events when used combination of rHuEPO and antiplatelet agents

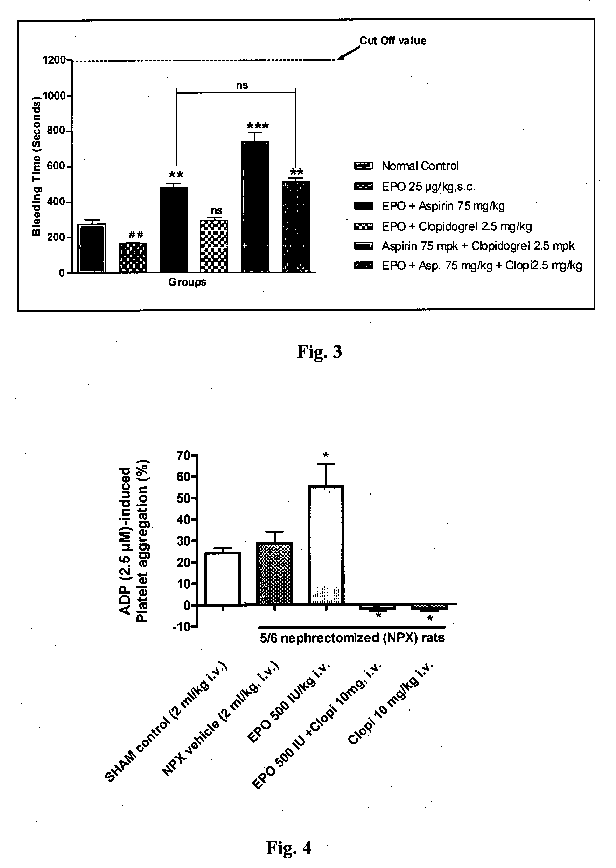

Six to eight week old male Wistar rats were divided in groups [each group containing 6 animals (n=6)]. Control animal received vehicle only (0.9 % w/v NaCl normal saline), treatment groups received three consecutive doses of Recombinant Human Erythropoietin (25 µg/kg, S.C.) at an interval of 24 hour. Aspirin (75mg/kg p.o.), and Clopidogrel (2.5mg/kg, p.o.) individually and in combination with each other were dosed to respective groups at 48 hour after the last injection of Erythropoietin. Bleeding time experiment using tail transaction test was performed. When clopidogrel and aspirin are used in combination, it increases the bleeding time as compared to normal. When rHuEPO is administered with combination of clopidogrel and aspirin, it significantly reduces this increased bleeding time in Wistar rats (Fig 3). The same effect is found with clopidogrel and aspirin, when administered individually with rHuEPO. Results are summarized in table 3.

**Table 3: Effect of rHuEPO on bleeding time, when combined with Clopidogrel and Aspirin**

| | Normal control | Clopidogrel (2.5 mpk) and Aspirin (75 mpk) | rHuEPO (25 µg/kg, s.c.) | rHuEPO (25 µg/kg, s.c.) and Aspirin (75 mpk) | rHuEPO (25 µg/kg, s.c.) and Clopidogrel (2.5 mpk) | rHuEPO (25 µg/kg, s.c.), Aspirin (75 mpk) and Clopidogrel (2.5 mpk) |
|---|---|---|---|---|---|---|
| Bleeding Time (in seconds) | 278 ± 33.4 | 740.0 ± 47.5 | 165 ± 28.1 | 485.0 ± 18.0 | 295.0 ± 18.0 | 511.7 ± 17.6 |

### Example 4: Reversal of platelet hyper-reactivity-induced by rHuEPO using combination of rHuEPO and antiplatelet agent in rat model of chronic kidney disease

Six to eight week old male Wistar rats used and the methods and procedures described here have been reviewed and approved by the Institutional Animal Ethics Committee (IAEC). We used human therapeutic dose of rHuEPO in rats and same frequency of administration as in humans *(*Kirkeby A et al., Thromb Haemost, 2008;99:720-728). A 5/6 nephrectomy has been performed on male wistar rats (Toba H et al., European Journal of Pharmacology 656, 2011, 81-87*)* and treatment has been started after one month of nephrectomy.
The rats were divided into 5 groups (n=6):
1) a control group, which received a sham operation (SHAM control);
2) a 5/6 nephrectomized group (NPX), removed the upper and lower poles of the left kidney and after one week the entire right kidney under anesthesia (NPX vehicle);
3) a 5/6 nephrectomy+rHuEPO group, nephrectomized and injected with rHuEPO at 500 IU/kg 3 times a week intravenously (i.v.) for 4 weeks (NPX+EPO);
4) a 5/6 nephrectomized+ rHuEPO+ clopidogrel group, nephrectomized and administered with rHuEPO (500 IU/kg) + clopidogrel (10 mg/kg) every 3 times a week intravenously (i.v.) for 4 weeks.
5) a 5/6 nephrectomized + clopidogrel group, nephrectomized and administered with rHuEPO (500 IU/kg) + clopidogrel (10 mg/kg) every 3 times a week intravenously (i.v.) for 4 weeks.

After 4 weeks of treatment, blood samples were collected via retro-orbital route in tubes containing 3.8% tri-sodium citrate for the collection of platelet rich plasma (PRP) for ADP-induced platelet aggregation studies. Blood samples were also collected for hematological analysis in tubes containing 5% EDTA. Serum samples were used for measurement of various kidney biomarkers such as creatinine, urea, uric acid, blood urea nitrogen and total protein.

Increase in platelet aggregatory response meaning increase in platelet reactivity when same concentration of aggregating agent is used. When constant concentration of aggregating agents like ADP (2.5 µM) used, platelet aggregation increases with rHuEPO When constant concentration of aggregating agents like ADP (2.25 µM) is used, on administration rHuEPO (500 IU/kg) + clopidogrel (10 mg/kg) every 3 times a week intravenously (i.v.) for 4 weeks in nephrectomized rats, platelet aggregatory response is reversed (Fig 4). Results are summarized in Table 4.

**Table 4:**

| **Ex vivo platelet aggregation using 2.5 µM ADP in 5/6 nephrectomized rats** | | |
|---|---|---|
| | **Platelet aggregation (%)** | |
| **Groups** | **Average** | **SEM** |
| SHAM control (2 ml/kg i.v.) | 24.2 | 2.4 |
| NPX vehicle (2 ml/kg, i.v.) | 28.7 | 5.5 |
| EPO 500 IU/kg i.v. | 55 | 10.7 |
| EPO 500 IU +Clopi 10mg, i.v. | -1.9 | 1.1 |
| Clopi 10 mg/kg i.v. | -2 | 1.2 |

### Example 5: rHuEPO improves hematological parameters in combination with antiplatelet agent as well as alone in rat model of chronic kidney disease

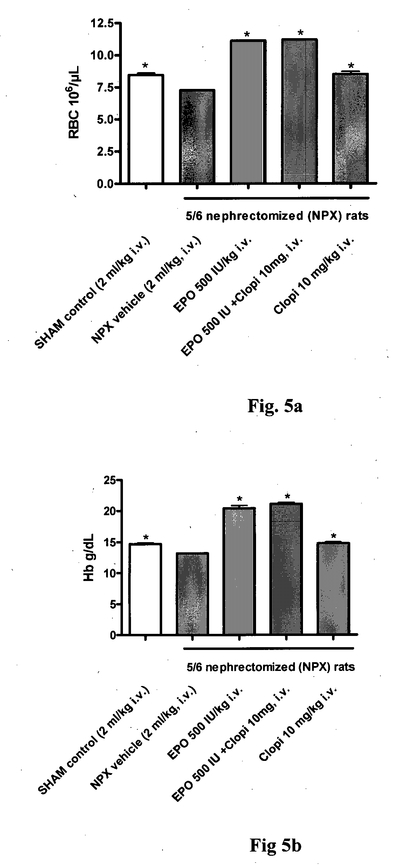

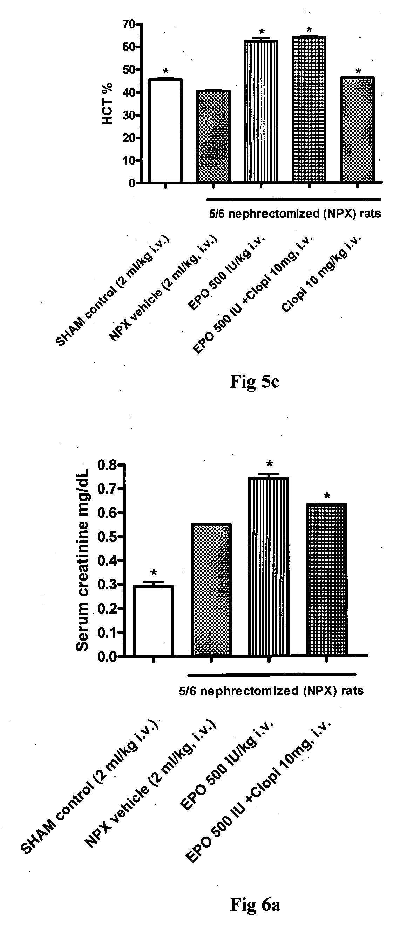

There was increase in RBC count, hemoglobin and hematocrit when rHuEPO is used alone and in combination with antiplatelet agent every 3 times a week intravenously (i.v.) for 4 weeks in nephrectomized rats (Fig 5a, 5b and 5c). Results are summarized in Table 5a, 5b and 5c.

**Table 5a:**

| **Effects of drug treatment on red blood cell (RBC) count in 5/6 nephrectomized rats** | | |
|---|---|---|
| | **RBC count (10⁶ µL)** | |
| **Groups** | **Average** | **SEM** |
| SHAM control (2 ml/kg i.v.) | 8.46 | 0.15 |
| NPX vehicle (2 ml/kg, i.v.) | 7.27 | 0.01 |
| EPO 500 IU/kg i.v. | 11.13 | 0.03 |
| EPO 500 IU +Clopi 10mg, i.v. | 11.2 | 0.06 |
| Clopi 10 mg/kg i.v. | 8.52 | 0.19 |

**Table 5b:**

| **Effects of drug treatment on hemoglobin (Hb) in 5/6 nephrectomized rats** | | |
|---|---|---|
| | **Hemoglobin (g/dL)** | |
| **Groups** | **Average** | **SEM** |
| SHAM control (2 ml/kg i.v.) | 14.68 | 0.21 |
| NPX vehicle (2 ml/kg, i.v.) | 13.17 | 0.03 |
| EPO 500 IU/kg i.v. | 20.4 | 0.47 |
| EPO 500 IU +Clopi 10mg, i.v. | 21.1 | 0.25 |
| Clopi 10 mg/kg i.v. | 14.77 | 0.22 |

**Table 5c:**

| **Effects of drug treatment on hematocrit (HCT) in 5/6 nephrectomized rats** | | |
|---|---|---|
| | **Hematocrit (%)** | |
| **Groups** | **Average** | **SEM** |
| SHAM control (2 ml/kg i.v.) | 45.52 | 0.57 |
| NPX vehicle (2 ml/kg, i.v.) | 40.6 | 0.36 |
| EPO 500 IU/kg i.v. | 62.3. | 1.45 |
| EPO 500 IU +Clopi 10mg, i.v. | 63.93 | 0.69 |
| Clopi 10 mg/kg i.v. | 46.07 | 0.52 |

### Example 6: rHuEPO treatment alone worsen the kidney function which has been evident by looking at the kidney biomarkers whereas rHuEPO in combination with antiplatelet agent improves the kidney function in rat model of chronic kidney disease

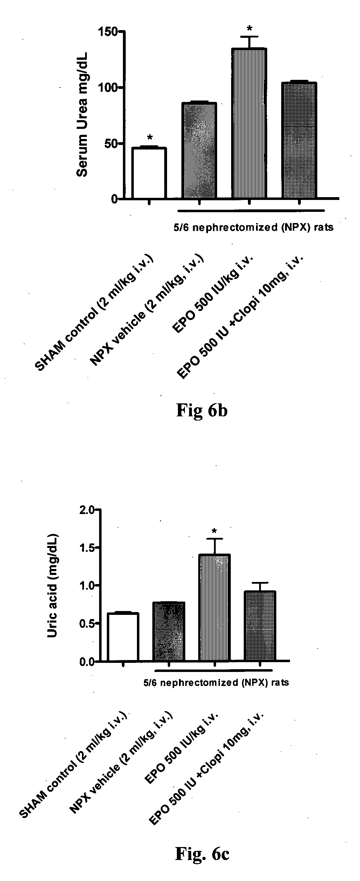

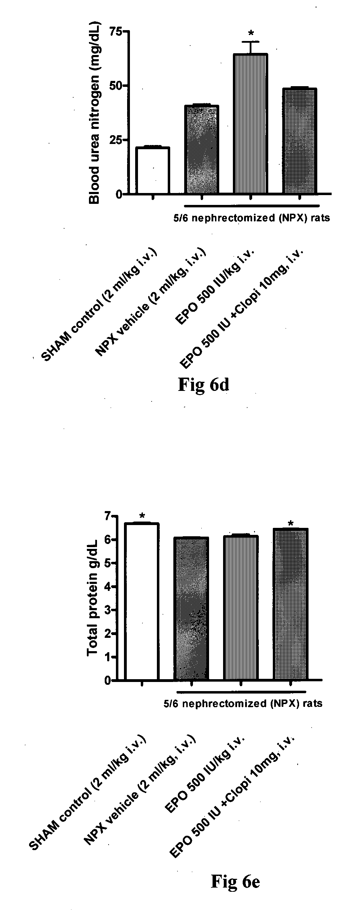

There was increase in the serum creatinine, serum urea, serum uric acid, blood urea nitrogen and decrease in serum total protein in nephrectomized animals. Surprisingly, there was further increase in the serum creatinine, serum urea, serum uric acid, blood urea nitrogen in nephretomized + rHuEPO treated rats which is the indicative of worsening the kidney function upon rHuEPO treatment. There was improvement in the kidney function when combination of rHuEPO and antiplatelet agent has been given every 3 times a week intravenously (i.v.) for 4 weeks in nephrectomized rats (Fig 6a, 6b, 6c, 6d and 6e). Results are summarized in Table 6a, 6b, 6c, 6d and 6e.

**Table 6a**

| **Effects of drug treatment on serum creatinine in 5/6 nephrectomized rats** | | |
|---|---|---|
| | **Serum creatinine mg/dL** | |
| **Groups** | **Average** | **SEM** |
| SHAM control (2 ml/kg i.v.) | 0.29 | 0.02 |
| NPX vehicle (2 ml/kg, i.v.) | 0.55 | 0.002 |
| EPO 500 IU/kg i.v. | 0.74 | 0.02 |
| EPO 500 IU +Clopi 10mg, i.v. | 0.63 | 0.002 |

**Table 6b**

| **Effects of drug treatment on serum urea in 5/6 nephrectomized rats** | | |
|---|---|---|
| | **Serum urea mg/dL** | |
| **Groups** | **Average** | **SEM** |
| SHAM control (2 ml/kg i.v.) | 45.8 | 1.56 |
| NPX vehicle (2 ml/kg, i.v.) | 85.67 | 1.76 |
| EPO 500 IU/kg i.v. | 134.33 | 10.81 |
| EPO 500 IU +Clopi 10mg, i.v. | 103.67 | 1.76 |

**Table 6c**

| **Effects of drug treatment on serum uric acid in 5/6 nephrectomized rats** | | |
|---|---|---|
| | **Serum uric acid mg/dL** | |
| **Groups** | **Average** | **SEM** |
| SHAM control (2 ml/kg i.v.) | 0.63 | 0.02 |
| NPX vehicle (2 ml/kg, i.v.) | 0.77 | 0.01 |
| EPO 500 IU/kg i.v. | 1.4 | 0.21 |
| EPO 500 IU +Clopi 10mg, i.v. | 0.91 | 0.12 |

**Table 6d**

| **Effects of drug treatment on blood urea nitrogen (BUN) in 5/6 nephrectomized rats** | | |
|---|---|---|
| | **Blood urea nitrogen mg/dL** | |
| **Groups** | **Average** | **SEM** |
| SHAM control (2 ml/kg i.v.) | 21.4 | 0.78 |
| NPX vehicle (2 ml/kg, i.v.) | 40.6 | 0.87 |
| EPO 500 IU/kg i.v. | 64.33 | 5.7 |
| EPO 500 IU +Clopi 10mg, i.v. | 48.33 | 0.88 |

**Table 6e**

| **Effects of drug treatment on total protein (TP) in 5/6 nephrectomized rats** | | |
|---|---|---|
| | **Total protein g/dL** | |
| **Groups** | **Average** | **SEM** |
| SHAM control (2 ml/kg i.v.) | 6.68 | 0.05 |
| NPX vehicle (2 ml/kg, i.v.) | 6.07 | 0.03 |
| EPO 500 IU/kg i.v. | 6.13 | 0.09 |
| EPO 500 IU +Clopi 10mg, i.v. | 6.43 | 0.03 |

## Claims

1. A composition for use in the treatment of kidney function in CKD patients comprising an antiplatelet agent or its pharmaceutically acceptable salt and an erythropoiesis stimulating agent.

2. The composition for use in the treatment of kidney function in CKD patients as claimed in claim 1 wherein the anti-platelet agents are selected from clopidogrel, aspirin, prasugrel or their suitable pharmaceutically acceptable salts.

3. The composition for use in the treatment of kidney function in CKD patients as claimed in claim 1 wherein pharmaceutically acceptable salt of clopidogrel are selected from besylate, hydrochloride, hydrobromide, mesylate, tosylate, bisulfate salts.

4. The composition for use in the treatment of kidney function in CKD patients as claimed in claim 1 wherein the anti-platelet agent is clopidogrel besylate.

5. The composition for use in the treatment of kidney function in CKD patients as claimed in claim 1 wherein erythropoiesis stimulating agent are selected from recombinant human erythropoietin, darbepoetin; PEG-recombinant human erythropoietin, and PEG-darbepoetin.

6. The composition for use in the treatment of kidney function in CKD patients as claimed in claim 1 comprising a therapeutic effective amount of clopidogrel or its pharmaceutically acceptable salts and recombinant human erythropoietin in a synergistic ratio.

7. The composition for use in the treatment of kidney function in CKD patients as claimed in any preceding claims wherein the therapeutic amount of clopidogrel or its pharmaceutically acceptable salts is selected from 5 to 300 mg.

8. The composition for use in the treatment of kidney function in CKD patients as claimed in any preceding claims wherein the therapeutic amount of clopidogrel is selected from 5 mg to 75 mg.

9. The composition for use in the treatment of kidney function in CKD patients as claimed in any preceding claims wherein the therapeutic amount of recombinant human erythropoietin is selected from 0.5µg/kg to 50µg/kg.

10. The composition for use in the treatment of kidney function in CKD patients as claimed in claim 9 wherein the therapeutic amount of recombinant human erythropoietin is selected from 0.5µg/kg to 2µg/kg.

11. The composition for use in the treatment of kidney function in CKD patients as claimed in any of the preceding claims wherein the antiplatelet agents are orally administrated.

12. The composition for use in the treatment of kidney function in CKD patients as claimed in any of the preceding claims wherein the antiplatelet agents are administrated by parenteral route.

## Patentansprüche

1. Zusammensetzung zur Verwendung in der Behandlung von Nierenfunktion bei CKD-Patienten, umfassend einen Thrombozytenhemmer oder das pharmazeutisch verträgliche Salz davon und ein erythropoiesestimulierendes Mittel.

2. Zusammensetzung zur Verwendung in der Behandlung von Nierenfunktion bei CKD-Patienten nach Anspruch 1, wobei die Thrombozytenhemmer aus Clopidogrel, Aspirin, Prasugrel oder die geeigneten pharmazeutisch verträglichen Salze davon ausgewählt sind.

3. Zusammensetzung zur Verwendung in der Behandlung von Nierenfunktion bei CKD-Patienten nach Anspruch 1, wobei das pharmazeutisch verträgliche Salz von Clopidogrel aus Besylat-, Hydrochlorid-, Hydrobromid-, Mesylat-, Tosylat-, Bisulfatsalzen ausgewählt ist.

4. Zusammensetzung zur Verwendung in der Behandlung von Nierenfunktion bei CKD-Patienten nach Anspruch 1, wobei der Thrombozytenhemmer Clopidogrelbesylat ist.

5. Zusammensetzung zur Verwendung in der Behandlung von Nierenfunktion bei CKD-Patienten nach Anspruch 1, wobei das erythropoiesestimulierende Mittel aus rekombinantem humanem Erythropoetin, Darbepoetin; PEGrekombinantem humanem Erythropoetin und PEG-Darbepoetin ausgewählt ist.

6. Zusammensetzung zur Verwendung in der Behandlung von Nierenfunktion bei CKD-Patienten nach Anspruch 1, umfassend eine therapeutische wirksame Menge an Clopidogrel oder die pharmazeutisch verträglichen Salze davon und rekombinantes humanes Erythropoetin in einem synergistischen Verhältnis.

7. Zusammensetzung zur Verwendung in der Behandlung von Nierenfunktion bei CKD-Patienten nach einem der vorgehenden Ansprüche, wobei die therapeutische Menge an Clopidogrel oder die pharmazeutisch verträglichen Salze davon von 5 bis 300 mg ausgewählt ist.

8. Zusammensetzung zur Verwendung in der Behandlung von Nierenfunktion bei CKD-Patienten nach einem der vorgehenden Ansprüche, wobei die therapeutische Menge an Clopidogrel von 5 mg bis 75 mg ausgewählt ist.

9. Zusammensetzung zur Verwendung in der Behandlung von Nierenfunktion bei CKD-Patienten nach einem der vorgehenden Ansprüche, wobei die therapeutische Menge von rekombinantem humanem Erythropoetin von 0,5 µg/kg bis 50 µg/kg ausgewählt ist.

10. Zusammensetzung zur Verwendung in der Behandlung von Nierenfunktion bei CKD-Patienten nach Anspruch 9, wobei die therapeutische Menge von rekombinantem humanem Erythropoetin von 0,5 µg/kg bis 2 µg/kg ausgewählt ist.

11. Zusammensetzung zur Verwendung in der Behandlung von Nierenfunktion bei CKD-Patienten nach einem der vorgehenden Ansprüche, wobei die Thrombozytenhemmer oral verabreicht werden.

12. Zusammensetzung zur Verwendung in der Behandlung von Nierenfunktion bei CKD-Patienten nach einem der vorgehenden Ansprüche, wobei die Thrombozytenhemmer parenteral verabreicht werden.

## Revendications

1. Composition pour l'utilisation dans le traitement de la fonction rénale chez les patients atteints d'IRC comprenant un agent antiplaquettaire ou son sel pharmaceutiquement acceptable et un agent stimulant l'érythropoïèse.

2. Composition pour l'utilisation dans le traitement de la fonction rénale chez les patients atteints d'IRC selon la revendication 1, dans laquelle les agents antiplaquettaires sont choisis parmi le clopidogrel, l'aspirine, le prasugrel ou leurs sels pharmaceutiquement acceptables et appropriés.

3. Composition pour l'utilisation dans le traitement de la fonction rénale chez les patients atteints d'IRC selon la revendication 1, dans laquelle un sel pharmaceutiquement acceptable de clopidogrel est choisi parmi les sels de bésylate, de chlorhydrate, de bromhydrate, de mésylate, de tosylate, de bisulfate.

4. Composition pour l'utilisation dans le traitement de la fonction rénale chez les patients atteints d'IRC selon la revendication 1, dans laquelle l'agent anti-plaquettaire est le clopidogrel bésylate.

5. Composition pour l'utilisation dans le traitement de la fonction rénale chez les patients atteints d'IRC selon la revendication 1, dans laquelle l'agent stimulant l'érythropoïèse est choisi parmi l'érythropoïétine humaine recombinante, la darbépoétine; l'érythropoïétine recombinante humaine de PEG et la darbépoétine de PEG.

6. Composition pour l'utilisation dans le traitement de la fonction rénale chez les patients atteints d'IRC selon la revendication 1, comprenant une quantité thérapeutique efficace du clopidogrel ou de ses sels pharmaceutiquement acceptables et de l'érythropoïétine humaine recombinante dans un rapport synergique.

7. Composition pour l'utilisation dans le traitement de la fonction rénale chez les patients atteints d'IRC selon l'une quelconque des revendications précédentes, dans laquelle la quantité thérapeutique du clopidogrel ou de ses sels pharmaceutiquement acceptables est choisie de 5 à 300 mg.

8. Composition pour l'utilisation dans le traitement de la fonction rénale chez les patients atteints d'IRC selon l'une quelconque des revendications précédentes, dans laquelle la quantité thérapeutique du clopidogrel est choisie entre 5 et 75 mg.

9. Composition pour l'utilisation dans le traitement de la fonction rénale chez les patients atteints d'IRC selon l'une quelconque des revendications précédentes, dans laquelle la quantité thérapeutique de l'érythropoïétine humaine recombinante est choisie de 0,5µg/kg à 50µg/kg.

10. Composition pour l'utilisation dans le traitement de la fonction rénale chez les patients atteints d'IRC selon la revendication 9, dans laquelle la quantité thérapeutique de l'érythropoïétine humaine recombinante est choisie de 0,5µg/kg à 2µg/kg.

11. Composition pour l'utilisation dans le traitement de la fonction rénale chez les patients atteints d'IRC selon l'une quelconque des revendications précédentes, dans laquelle les agents antiplaquettaires sont administrés par voie orale.

12. Composition pour l'utilisation dans le traitement de la fonction rénale chez les patients atteints d'IRC selon l'une quelconque des revendications précédentes, dans laquelle les agents antiplaquettaires sont administrés par voie parentérale.
